# EUROPÄISCHE PATENTANMELDUNG

(11) **EP 2 371 341 A1**
(43) Veröffentlichungstag der Anmeldung: **05.10.2011**
(21) Anmeldenummer: 11159246.5
(22) Anmeldetag: 22.03.2011
(51) Int. Cl.: A61J 1/06, A61M 5/24

(54) **Austragvorrichtung für Flüssigkeiten**

(30) Priorität: 26.03.2010 DE 102010013543
(71) Anmelder: Ing. Erich Pfeiffer GmbH, 78315 Radolfzell (DE)
(72) Erfinder: Greiner-Perth, Jürgen, 78244, Gottmadingen (DE); Ruf, Alexander, 78345, Moos-Bankholzen (DE)
(74) Vertreter: Patentanwälte Ruff, Wilhelm, Beier, Dauster & Partner

(57) **Zusammenfassung**

1. Austragvorrichtung für pharmazeutische Flüssigkeiten.
2.1. Die Erfindung betrifft eine Austragvorrichtung für pharmazeutische Flüssigkeiten mit einer Betätigungseinheit (40) zur manuellen Bewirkung eines Austragvorgangs und einem Flüssigkeitsspeicher (20), in dem die pharmazeutische Flüssigkeit vor dem Austragvorgang gelagert ist, wobei eine erste Teileinheit (50) der Betätigungseinheit (40) mit einer Auslassöffnung (52b) versehen ist und wobei eine zweite Teileinheit (60) der Betätigungseinheit (40) zur Bewirkung des Austragvorgangs gegenüber der ersten Teileinheit (50) verlagerbar ist.
2.2. Erfindungsgemäß, ist vorgesehen, dass die beiden Teileinheiten (50, 60) der Betätigungseinheit (40) gemeinsam einen Aufnahmeraum (70) begrenzen, von dem aus ein Flüssigkeitskanal (54a) zur Auslassöffnung (52b) führt, dass der Flüssigkeitsspeicher (20) zur Anordnung im Aufnahmeraum (70) vorgesehen und als Flüssigkeitsbeutel (20) ausgebildet ist, dessen Wandungen als Folienwandungen ausgebildet sind, die einen mit der Flüssigkeit befüllten Innenraum begrenzen, und dass die Betätigungseinheit (40) und der Flüssigkeitsbeutel (20) derart aufeinander abgestimmt sind, dass durch eine Relativverlagerung der Teileinheiten (50, 60) der Betätigungseinheit (40) zueinander ein Öffnen des Flüssigkeitsbeutels (20) und eine Volumenreduzierung des Aufnahmeraums (70) mit daraus resultierender Volumenreduzierung des Flüssigkeitsbeuteis (20) und ein Ausbringung der Flüssigkeit (38) durch die Auslassöffnung (52b) bewirkt wird.

## Beschreibung

### Anwendungsgebiet und Stand der Technik

Die Erfindung betrifft eine Austragvorrichtung für pharmazeutische Flüssigkeiten mit einer Betätigungseinheit zur manuellen Bewirkung eines Austragvorgangs und mit einem Flüssigkeitsspeicher, in dem die pharmazeutische Flüssigkeit vor dem Austragvorgang gelagert ist. Dabei ist eine erste Teileinheit der Betätigungseinheit mit einer Auslassöffnung versehen und eine zweite Teileinheit der Betätigungseinheit zur Bewirkung des Austragvorgangs gegenüber der ersten Teileinheit verlagerbar,

Eine solche Austragvorrichtung ist beispielsweise aus der DE 44 12 041 A1 bekannt. Diese gattungsgemäße Austragvorrichtung ist zur einmaligen Verwendung vorgesehen. Sie verfügt über zwei gegeneinander verschiebbare Teileinheiten, die jeweils über Fingerauflageflächen verfügen. Durch Zusammendrücken der Teileinheiten kann ein Austragvorgang bewirkt werden, im Zuge dessen ein mittels eines Gummistopfens verschlossene Glaszylinder durch Durchstechen des Gummistopfens geöffnet wird und der Gummistopfen anschließend in den Glaszylinder eingeschoben wird, so dass hierdurch in der Art einer Schubkolbenpumpe die Flüssigkeit aus dem Glaszylinder durch die Nadel hindurch ausgetragen werden kann.

Die aus dem Stand der Technik bekannten Austragvorrichtungen gattungsgemäßer Art haben sich insbesondere für Impfzwecke bewährt. Als nachteilig werden allerdings die vergleichsweise hohen Kosten angesehen, die maßgeblich durch den Glaszylinder und dem Gummistopfen verursacht werden. Diese vergleichsweise hohen Kosten führen dazu, dass gattungsgemäße Austragvorrichtungen der beschriebenen Art bislang vor allem für vergleichsweise hochpreisige Medikamente Verwendung fanden.

### Aufgabe und Lösung

Aufgabe der Erfindung ist es, eine gattungsgemäße Austragvorrichtung dahingehend weiterzubilden, dass diese bei hoher Zuverlässigkeit günstig hergestellt werden kann.

Erfindungsgemäß wird dies dadurch erreicht, dass die beiden Teileinheiten der Betätigungseinheit gemeinsam einen Aufnahmeraum begrenzen, von dem aus ein Flüssigkeitskanal zur Auslassöffnung führt. Dieser Aufnahmeraum dient der Aufnahme eines Flüssigkeitsspeichers, welcher als Flüssigkeitsbeutel ausgebildet ist. Die Wandungen dieses Flüssigkeitsbeutels sind als Folienwandungen ausgebildet und begrenzen einen mit der Flüssigkeit befüllten Innenraum. Die Betätigungseinheit und der Flüssigkeitsbeutel sind dabei derart aufeinander abgestimmt, dass durch eine Relativverlagerung der Teileinheit der Betätigungseinheit zueinander ein Öffnen des Flüssigkeitsbeutels sowie eine Volumenreduzierung des Aufnahmeraums mit daraus resultierender Volumenreduzierung des Flüssigkeitsbeutels und dadurch einer Ausbringung der Flüssigkeit durch die Auslaßöffnung bewirkt werden kann.

Die beiden Teileinheiten der Betätigungseinheit sind so aufeinander abgestimmt, dass sie in einer definierten Richtung oder entlang eines definierten Pfades gegeneinander verlagert werden können, um das Innenvolumen des Aufnahmeraums zu reduzieren. Vorzugsweise sind die Teileinheiten durch Führungsmittel translativ gegeneinander geführt und verlagerbar. Zur Kraftbeaufschlagung der Teileinheiten sind beide Teileinheiten vorzugsweise mit Fingerauflageflächen versehen, wobei insbesondere vorzugsweise die erste Teileinheit zwei Fingerauflagen für Zeigefinder und Mittelfinger aufweist und die zweite Teileinheit eine Fingerauflage für den Daumen aufweist.

Im Bereich der Auslassöffnung ist vorzugsweise eine Zerstäubungsgeometrie angeordnet, die der Zerstäubung der Flüssigkeit dient. Als insbesondere vorteilhaft wird es angesehen, wenn die Austragvorrichtung zur nasalen Applikation vorgesehen ist und hierfür- eine konische Nasenolive aufweist, die zur Einführung in ein Nasenloch ausgebildet ist.

Die beiden Teileinheiten der Betätigungseinheit sind in einem Lieferzustand vorzugsweise voneinander getrennt und werkzeuglos miteinander verbindbar, wobei insbesondere eine Teileinheit, vorzugsweise die zweiten Teileinheit, in die andere Teileinheit, vorzugsweise die erste Teileinheit, einsteckbar ist. Die werkzeuglose und daher für den Anwender durchführbare Verbindbarkeit der Teileinheiten gestattet die Einfügung des Flüssigkeitsspeichers in den Aufnahmeraum unmittelbar vor Ausbringung der Flüssigkeit. Dies erlaubt es insbesondere, die gegebenenfalls kühlbedürftigen Flüssigbeutel getrennt von der nicht kühlbedürftigen Betätigungseinheit zu lagern.

Der Flüssigkeitsspeicher selbst ist als Flüssigkeitsbeutel ausgebildet und daher vergleichsweise preiswert herstellbar, Er wird vor dem Zusammenfügen der Teileinheiten der Betätigungseinheit im Aufnahmeraum platziert. Wenn die Teileinheiten der Betätigungseinheit gegeneinander verlagert werden, bewirkt dies auf nachfolgend noch erläuterte Weise ein Öffnen des Flüssigkeitsspeichers, so dass das ausströmende Medium über den Flüssigkeitskanal zur Auslassöffnung gelangen kann. Bei fortschreitender Verlagerung der Betätigungseinheiten gegeneinander wird der geöffnete Flüssigkeitsbeutel zusammengedrückt. Er wird dadurch gleichsam ausgepresst und die Flüssigkeit wird in den Flüssigkeitskanal gedrückt, der zur Auslassöffnung führt. Der Aufnahmeraum ist vorzugsweise zylindrisch ausgebildet und an einer Seite durch eine Kolbenfläche begrenzt, wobei vorzugsweise die Zylinderinnenfläche an der ersten Teileinheit und die Kolbenfläche an der zweiten Teileinheit angebracht sind. Damit das Medium nicht auf anderem Wege in die die Austragvorrichtung umgebende Atmosphäre gelangt, ist ein Kontaktbereich, in dem die beiden Teileinheiten aneinander anliegen, vorzugsweise flüssigkeitsdicht ausgebildet.

je nach Gestaltung des Aufnahmeraums kann dieser geeignet sein, den Flüssigkeitsbeutel in beliebiger Orientierung aufzunehmen, so dass ein besonders unkompliziertes Einfügen des Flüssigkeitsbeutels ermöglicht wird.

Bevorzugt ist allerdings eine Abstimmung des Flüssigkeitsbeutels und des Aufnahmeraums, durch die eine oder zwei mögliche Orientierungen des Flüssigkeitsbeutels im Aufnahmeraum vorgegeben werden. Besonders von Vorteil ist es, wenn der Flüssigkeitsbeutel eine längliche Form aufweist und im eingesetzten Zustand parallel zur Verlagerungsrichtung der Teileinheiten ausgerichtet ist. Hierdurch ist besonders reproduzierbar eine weitgehend vollständige Ausbringung der Flüssigkeit möglich, da der Flüssigkeitsbeutel in Richtung seiner maximalen Erstreckung komprimiert wird. Da die längliche Gestaltung des Flüssigkeitsbeutels zwei mögliche Einsetzrichtungen schafft, von denen gegebenenfalls eine als bevorzugt angesehen wird, kann vorgesehen sein, dass auf dem Flüssigkeitsbeutel eine Markierung vorgesehen ist, die die bevorzugte Orientierung kennzeichnet.

Bezogen auf eine kreisförmige Querschnittsfiäche beträgt der mittlere Durchmesser des Innenraums des Flüssigkeitsbeutels vorzugsweise zwischen 3 mm und 10 mm, insbesondere zwischen 4 mm und 6 mm. Die Länge des Innenraums des Flüssigkeitsbeutels beträgt vorzugsweise zwischen 10 mm und 30 mm, insbesondere zwischen 15 mm und 25 mm. Für die zum Austrag mit der erfindungsgemäßen Austragvorrichtung vorgesehenen pharmazeutischen Flüssigkeiten wird es als bevorzugt angesehen, wenn ein Volumen des Innenraums des Flüssigkeitsbeutels im ungeöffneten Zustand unter 1000 µl beträgt, vorzugsweise zwischen 100 µl und 500 µl.

Die den Innenraum des Flüssigkeitsbeutels umgebende Folienwandung ist vorzugsweise von einheitlicher Art. Vorzugsweise sind somit keine Folienabschnitte unterschiedlichen Typs vorgesehen, die verschiedene Wandungsabschnitte der Folienwandung bilden, Dies gestattet es, die Folienwandungen des Flüssigkeitsbeutels aus nur einem Typ von jedoch gegebenenfalls mehrlagigem Folienmaterial herzustellen, vorzugsweise insbesondere aus einem einstückigen Folienabschnitt, der umgeschlagen wird und dessen dadurch entstandenen zwei Lagen randseitig miteinander verbunden, insbesondere verschweißt, werden. Insbesondere kann der Flüssigkeitsbeutel in einem kontinuierlichen Verfahren als Schlauchbeutel hergestellt werden. Die Gestaltung als Schlauchbeutel führt zu einer sehr einfachen und sehr schnellen Herstellbarkeit einer Vielzahl von Flüssigkeitsbeuteln. Dies ist insbesondere im Zusammenhang mit Impfstoffen von erheblicher Vorteil, die häufig kurzfristig in großer Menge benötigt werden.

Bei einer Herstellung des Flüssigkeitsbeutels als kontinuierlich hergestellter Schlauchbeutel werden die Folienwandungen vorzugsweise durch eine einmal umgeschlagene Folienbahn gebildet, deren Seitenränder miteinander durch eine Längsnaht verbunden werden, so dass eine Schlauchform erzielt wird. Voneinander isoliert werden die Innenräume der späteren Flüssigkeitsbeutel durch Quernähte, die den Schlauch in Einzelkanimern und somit in Flüssigkeitsbeutel unterteilen. Diese zusammenhängenden Flüssigkeitsbeutel werden anschließend voneinander getrennt oder zum Zwecke der späteren Trennung im Bereich der Quernähte mit einer Sollrisslinie, wie beispielsweise einer Perforation versehen.

Die Folienwandung ist vorzugsweise derart beschaffen, dass die Komprimierung des Flüssigkeitsbeutels zu keinen relevanten Rückstellkräften (< 5 Newton) im Zuge der Verformung der Folienwandung führt. Dies kann durch eine plastische Verformbarkeit der Folienwandung gewährleistet sein, wie sie beispielsweise bei einer Metallfolie oder einer Folie mit einer Metalllage gegeben ist. Auch eine leicht verformbare Weichfolie ist geeignet. Die Verwendung einer selbsttragenden und durch Thermoformung/Tiefziehen in eine dreidimensionale Form gebrachten Hartfolie wird dagegen als nachteilig angesehen. Die Dicke der Folie beträgt vorzugsweise weniger als 150 µm, insbesondere vorzugsweise weniger als 100 µm.

Insbesondere von Vorteil ist es, wenn die Folienwandung des Flüssigkeitsbeutels diffusionsdicht ausgebildet ist. Da die Flüssigkeitsmenge im Flüssigkeitsbeutel sehr gering ist und die Lagerzeiten mitunter vergleichsweise lang sein können, ist eine solche diffusionsdichte Gestaltung erwünscht, um auch langfristig die Ausbringung der wunschgemäßen Menge der Flüssigkeit gewährleisten zu können. Eine Möglichkeit zur Gestaltung der Folienwandung sieht vor, dass diese eine Beschichtung, beispielsweise aus Keramik oder Metall, insbesondere aus Aluminium, erhält, Diese Beschichtung kann beispielsweise auf eine Kunststoffträgerfolie aufgedampft werden. Alternativ kann die Folienwandung selbst oder eine Schicht der Folienwandung aus Metall, insbesondere Aluminium, oder aus einem besonders diffusionsdichten Kunststoff, insbesondere aus Ethylen-Vinyl-Alkohol-Copolymer (EVOH), gebildet sein.

Hinsichtlich des Öffnens des Flüssigkeitsbeutels im Zuge der Betätigung sind insbesondere zweierlei Gestaltungen zweckmäßig.

Es kann vorgesehen sein, dass die Betätigungseinheit und der Flüssigkeitsbeutel derart aufeinander abgestimmt sind, dass durch eine Relativverlagerung der beiden Teileinheiten der Betätigungseinheit eine Druckerhöhung im Flüssigkeitsbeutel bewirkt werden kann, die bei ausreichender Kraftbeaufschlagung der Teileinheiten gegeneinander ein Platzen des Flüssigkeitsbeutels verursacht, insbesondere im Bereich einer Folienwandung oder einer Schweißnaht. Bei einer solchen Gestaltung erfolgt das Öffnen des Flüssigkeitsbeutels somit nur mittelbar über die Verfeinerung des Aufnahmeraums und unmittelbar über die Druckerhöhung in der Flüssigkeit. Der Flüssigkeitsbeutel kann zur Erzielung einer reproduzierbaren Öffnungscharakteristik mit gezielt angeordneten Schwachstellen ausgebildet sein. So ist es insbesondere möglich, den Flüssigkeitsbeutel mit einer gegenüber den anderen Schweißnähten schwächeren Schweißnaht zu versehen, die in Richtung des Flüssigkeitskanals gerichtet ist.

Alternativ hierzu kann die Betätigungseinheit ein scharfkantiges Öffnungsmittel zum Öffnen des Flüssigkeitsbeutels aufweisen. Bei einer solchen Gestaltung ist somit vorgesehen, dass das Öffnen des Flüssigkeitsbeutels nicht primär durch den Druck der Flüssigkeit bewirkt wird, sondern durch den Kontakt des Flüssigkeitsbeutels mit dem scharfkantigen Öffnungsmittel und einem dadurch verursachten Punktieren oder Aufschneiden des Flüssigkeitsbeutels. Hierdurch ist ein präzises Festlegen des Ortes, an dem der Flüssigkeitsbeutel geöffnet wird, möglich.

Im einfachsten Fall kann das vorzugsweise metallische Öffnungsmittel als einfache Schneide oder Nadel ausgebildet sein, die in den Aufnahmeraum hineinragt.

Von besonderem Vorteil ist es jedoch, wenn das Öffnungsmittel durch eine an ihrem distalen Ende offene und mit dem Flüssigkeitskanal verbundene Hohlnadel gebildet ist. Diese Hohlnadel dient somit nicht nur dem Öffnen des Flüssigkeitsbeutels, sondern bildet zugleich den Kanal, durch den die Flüssigkeit aus dem Flüssigkeitsbeutel in den Flüssigkeitskanal der ersten Teileinheit gelangen kann. Hierdurch wird bewirkt, dass zumindest ein Großteil der Flüssigkeit nicht in den den Flüssigkeitsbeutel umgebenden Teil des Aufnahmeraums gelangt.

Besonders von Vorteil ist es, wenn die Hohlnadel sich zu ihrem distalen Ende hin verjüngt, da hierdurch die Gefahr verringert wird, dass relevante Mengen der Flüssigkeit an der Hohlnadel vorbei aus dem Flüssigkeitsbeutel in den Aufnahmeraum gelangen. Weiterhin ist es von Vorteil, wenn die Hohlnadel seitlich, also im Bereich ihrer Mantelfläche, mindestens eine weitere Öffnung aufweist, wobei diese vorzugsweise als längs erstreckter Schlitz ausgebildet ist. In Falle der schlitzartigen Gestaltung kann die zusätzliche Öffnung auch mit der distalen Hauptöffnung der Hohlnadel zusammenhängen. Durch die weitere Öffnung wird zum einen bewirkt, dass an der Hohlnadel vorbei in den Aufnahmeraum gelangte Flüssigkeit zurück in die Hohlnadel und damit zur Auslassöffnung gelangen kann. Des Weiteren ist eine solche Gestaltung von Vorteil, wenn die Hohlnadel bestimmungsgemäß im Zuge des Austragens mit ihrem distalen Ende die Folienwandung gegenüberliegend zur Öffnungsstelle nochmals durchdringt, da die Flüssigkeit aus dem Flüssigkeitsbeutel in einem solchen Falle auch nach dieser zweiten Durchdringung der Folienwandung in die Hohlnadel hinein und zur Auslassöffnung gelangen kann.

Der sich verjüngende Teilabschnitt der Hohlnadel und/oder die zusätzliche Öffnung erstrecken sich vorzugsweise über einen Bereich von mindestens 3 mm, insbesondere mindestens 5 mm.

Dem Öffnungsmittel ist vorzugsweise ein gegenüber dem Öffnungsmittel relativ verlagerbarer Schutzabschnitt zugeordnet, der in einer Sicherungsstellung verhindert, dass das Öffnungsmittel mit dem Flüssigkeitsbeutel in Kontakt gelangt, und der in einer Funktionsstellung das Öffnungsmittel freigibt. Dieser Schutzabschnitt, der vorzugsweise eine verlagerbare Wandung des Aufnahmeraums bildet, kann somit während des Betätigens zunächst ein Öffnen des Flüssigkeitsbeutels verhindern, so dass in dieser ersten Phase nur eine Druckerhöhung im Flüssigkeitsbeutel bewirkt wird. Erst durch eine vorzugsweise durch die Relativverlagerung der Teileinheiten bewirkte Relativverlagerung des Schutzabschnitts gegenüber dem Öffnungsmittel wird das Öffnungsmittel in Kontakt mit dem Flüssigkeitsbeutel gebracht und bewirkt das Öffnen des Flüssigkeitsbeutels.

Vorzugsweise ist der Schutzabschnitt derart angeordnet und/oder ausgebildet, dass er durch eine Kraftbeaufschlagung der Teileinheiten der Betätigungseinheit aus der Sicherungsstellung in die Funktionsstellung verlagert wird. Dabei ist insbesondere vorzugsweise vorgesehen, dass diese Kraftbeaufschlagung über den Flüssigkeitsbeutel auf den Schutzabschnitt übertragen wird. Der Schutzabschnitt wird somit durch den Flüssigkeitsbeutel selbst verlagert, so dass anhand der Stabilität der Anbringung des Schutzabschnitts in dessen Sicherungsstellung festgelegt werden kann, bei welchem Druck im Flüssigkeitsbeutel das Öffnen erfolgt. Hierdurch kann ein hoher Druck im Flüssigkeitsbeutel zum Zeitpunkt des Öffnens gewährleistet werden, der verhindert, dass Flüssigkeitsreste in Falten des Flüssigkeitsbeutels verbleiben, die nicht ausgetragen werden können, und der weiterhin die gewünschte Austragcharakteristik gewährleistet.

Vorzugsweise sind die Betätigungseinheit und/oder der Flüssigkeitsbeutel derart ausgebildet und aufeinander abgestimmt, dass ein Öffnen des Flüssigkeitsbeutels bewirkt wird, wenn die Teileinheiten mit einer Betätigungskraft von mindestens 10 Newton, vorzugsweise einer Betätigungskraft zwischen 10 Newton und 30 Newton, gegeneinander gedrückt werden. Somit wird ein für den Benutzer fühlbarer Druckpunkt erzeugt, der auch einem versehentlichen Bewirken eines Austragvorgangs entgegen wirkt. Die genannte Betätigungskraft reicht vorzugsweise aus, um nach Überwindung des Druckpunktes für den Benutzer nahezu unvermeidbar einen vollständigen Austrag der Flüssigkeit aus dem Flüssigkeitsbeutel zu bewirken. Der Betätigungskraft ist vorzugsweise derart ausgelegt, dass ein Austragdruck zwischen 5 und 6 bar erzielt wird.

Im Falle einer Gestaltung mit Schutzabschnitt wird es als besonders bevorzugt angesehen, wenn der Schutzabschnitt bis zum Erreichen dieser Betätigungskraft mittels mindestens eines als Sollbruchstelle agierenden Sicherungsabschnitts in seiner Sicherungsstelle gehalten wird. Dieser Sicherungsabschnitt versagt, sobald die genannte Betätigungskraft erreicht wird, und gestattet dann die Verlagerung des Schutzabschnitts in dessen Funktionsstellung und damit das Öffnen des Flüssigkeitsbeutels.

Die Erfindung betrifft weiterhin einen Flüssigkeitsbeutel für eine Austragvorrichtung der beschriebenen Art. Dieser Flüssigkeitsbeutel weist Wandungen auf, die als Folienwandungen ausgebildet sind und die einen mit Flüssigkeit befüllten Innenraum begrenzen. Dieser mit pharmazeutischer Flüssigkeit befüllte Innenraum weist ein Volumen von maximal 1000 µl auf. Insbesondere weist er ein Volumen zwischen 100 µl und 500 µl auf.

Die im Flüssigkeitsbeutel gelagerte pharmazeutische Flüssigkeit ist insbesondere als Impfstoff oder als Schmerzmittel ausgebildet. Insbesondere für solche pharmazeutischen Flüssigkeiten wird die Verwendung einer erfindungsgemäßen Austragvorrichtung als vorteilhaft angesehen.

Der Flüssigkeitsbeutel weist insbesondere vorzugsweise die oben bereits genannten Maße auf. Er ist in oben beschriebener Art vorzugsweise als Schlauchbeutel ausgebildet.

Die Erfindung betrifft auch eine Mehrzahl von solchen Flüssigkeitsbeuteln, die einen zusammenhängenden Streifen bilden, wobei vorzugsweise zwischen den einzelnen Flüssigkeitsbeuteln werkzeuglos trennbare Perforationen vorgesehen sind. In Form eines solchen Streifens, der vorzugsweise in Form eines Wickels vorgehalten wird, können die Flüssigkeitsbeutel auf engstem Raume gelagert und gekühlt werden.

### Kurzbeschreibung der Zeichnungen

Weitere Aspekte und Vorteile der Erfindung ergeben sich außer aus den Ansprüchen auch aus der nachfolgenden Beschreibung zweier bevorzugter Ausführungsbeispiele der Erfindung, die anhand der Figuren erläutert werden. Dabei zeigen:
- Fig. 1a und 1b: Flüssigkeitsspeicher zur Verwendung mit einer erfindungsgemäßen Austragvorrichtung,
- Fig. 2a bis 2d: eine erste Ausführungsform einer erfindungsgemäßen Austragvorrichtung in verschiedenen Stadien des Austragvorgangs und
- Fig. 3a bis 3d: eine zweite Ausführungsform einer erfindungsgemäßen Austragvorrichtung in verschiedenen Stadien des Austragvorgangs.

### Detaillierte Beschreibung der Ausführungsbeispiele

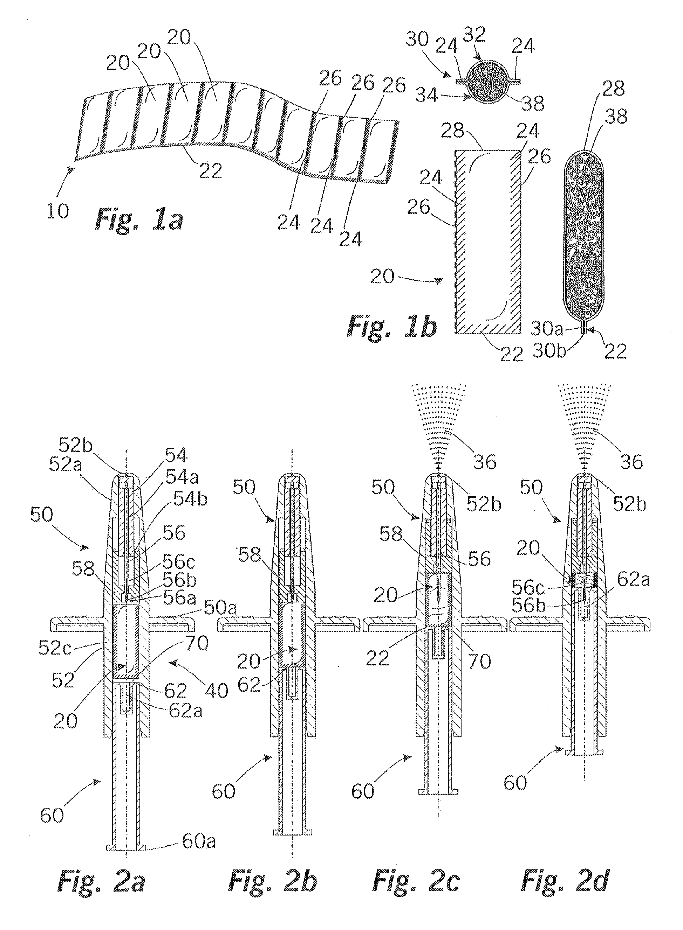

Fig. 1a zeigt eine Vielzahl von Flüssigkeitsbeuteln 20 für eine erfindungsgemäße Austragvorrichtung. Diese Flüssigkeitsbeutel 20 sind durch ein kontinuierliches Verfahren als Schlauchbeutel hergestellt. Sie bilden daher einen zusammenhängenden Streifen 10, der in einem Lieferzustand vorzugsweise wickelartig aufgerollt ist. In Fig. 1b ist ein Flüssigkeitsbeutel 20 in einer Einzelansicht sowie geschnitten dargestellt

Die Wandungen 30 der Flüssigkeitsbeutel 20 sind aus einer Folienbahn hergestellt, die zunächst einmal umgeschlagen wurde, um dann mittels einer Längsschweißnaht 22 an ihren zuvor gegenüberliegenden Kanten verbunden zu werden. Mittels Querschweißnähten 24 werden bei der Herstellung die Innenräume der einzelnen Flüssigkeitsbeutel 20 voneinander getrennt. Im Bereich der Quernähte 24 wird bei der Herstellung weiterhin eine Perforationslinie 26 vorgesehen, die die werkzeuglose Trennung der Flüssigkeitsbeutel 20 voneinander ermöglicht.

Wie in Fig. 1b dargestellt weisen die Flüssigkeitsbeutel 20 eine längliche Gestalt auf und sind mit einer pharmazeutischen Flüssigkeit 38, beispielsweise einem Impfstoff, befüllt. Die Wandung 30 des Flüssigkeitbeutels 20 weist eine Vorderseite 32 und eine Rückseite 34 auf, welche im Bereich einer Umschlagkante 28 direkt miteinander verbunden sind. In Längsrichtung des Flüssigkeitsbeutels 20 sind die Vorderseite 32 und Rückseite 34 mittels der Quernähte 24 miteinander verbunden. Auf der der Umschlagkante 28 gegenüberliegenden Seite sind die Vorderseite 32 und die Rückseite 34 durch die Längsnaht 22 miteinander verbunden.

Die Folienwandung 30 weist einen Schichtaufbau mit einer äußeren Trägerschicht 30a aus Kunststoff und einer inneren Schicht 30b aus Aluminium auf. Die Aluminiumschicht 30b führt zu einer hohen Dichtigkeit, so dass die Menge der Flüssigkeit 38 sich auch bei langzeitiger Lagerung nicht durch Diffusion verringert. Es können auch weitere Wandungsschichten vorgesehen sein, insbesondere eine zusätzliche Kunststoffschicht an der innenseite der Aluminiumschicht 30b.

Der Innenraum, in dem die pharmazeutische Flüssigkeit 38 angeordnet ist, weist eine Länge von 20 mm und einen Durchmesser von etwa 4 mm auf. Das Volumen des Innenraums beträgt etwa 220 µl.

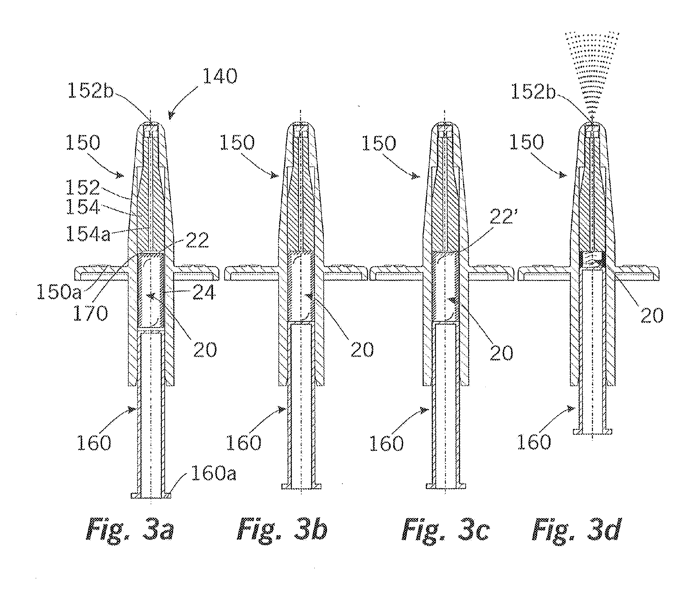

Die Verwendung eines Flüssigkeitsbeutels gemäß der Fig. 1a und 1b wird nachfolgend anhand der Fig. 2a bis 2d und 3a bis 3d erläutert.

Fig. 2a bis 2d zeigen eine erste Ausführungsform einer Austragvorrichtung zur Ausbringung der Flüssigkeit 38 aus einem Flüssigkeitsbeutel 20. Diese Austragvorrichtung weist neben dem Flüssigkeitsbeutel eine Betätigungseinheit 40 auf. Diese Betätigungseinheit 40 wiederum weist eine erste Teileinheit 50 und eine gegenüber der ersten Teileinheit 50 bestimmungsgemäß, manuell verlagerbare zweite Teileinheit 60 auf.

Die erste Teileinheit 50 besteht aus einem hülsenartigen Bauteil 52, an dessen oberen Ende eine Nasenolive 52a vorgesehen ist. Das Ende der Nasenolive 52a wird durch eine Auslassöffnung 52b gebildet. Am gegenüberliegenden Ende weist das Bauteil 52 einen hohlzylindrischen Abschnitt 52c auf, der zur Aufnahme der zweiten "l"eileinheit 60 vorgesehen ist. Innerhalb des hülsenförmigen Bauteils 52 weist die erste Teileinheit 50 einen aus produktionstechnischen Gründen separaten, jedoch mit dem Bauteil 52 fest verbundenen Einsatz 54 auf. In diesem ist ein Kanal 54a vorgesehen, der zur Auslassöffnung 52b führt, und in den eine Hohlnadel 56 fest eingefügt ist. Verschiebbar auf der Hohlnadel 56 und dem Einsatz 54 angeordnet ist ein Schutzabschnitt 58, der die Hohlnadel 56 in der Sicherungsstellung der Fig. 2a nach unten hin überragt. Eine Verlagerung des Schutzabschnitts 58 relativ zu den Bauteilen 52, 54 nach oben wird durch einen am Einsatz 54 angeformten Sicherungsring 54b zunächst verhindert.

Die Hohlnadel 56, die in den Einsatz 54 eingepresst ist, weist an ihrem nach unten weisenden Ende eine scharfkantige Spitze 56a und eine Zugangsöffnung 56b auf. Weiterhin ist die Hohlnadel 56 von der Öffnung 56b bis zum Einsatz 54 mit einem seitlichen Längsschlitz 56c versehen, durch den hindurch Flüssigkeit ebenfalls in die Hohlnadel 56 hineingelangen kann.

Die zweite Teileinheit 60 ist als vergleichsweise einfaches hohlzylindrisches Bauteil gestaltet, an dessen oberen Ende eine Kolbenfläche 62 vorgesehen ist. Mittig ist in dieser Kolbenfläche 62 eine Vertiefung 62a zur Aufnahme der Hohlnadel 56 im Zuge der Betätigung vorgesehen.

In dem in Fig. 2a dargestellten Zustand ist die zweite Teileinheit 60 bereits in den hohlzylindrischen Abschnitt 52c der ersten Teileinheit 50 eingeschoben. Gemeinsam begrenzen die Teileinheiten 50, 60 durch die innenwandung des hohlzylindrischen Abschnitts 52c, die Endfläche des Schutzabschnitts 58 und die Kolbenfläche 62 einen Aufnahmeraum 70, in dem ein Flüssigkeitsbeutel 20 der beschriebenen Art eingesetzt ist. Dabei ist dieser Flüssigkeitsbeutel derart eingesetzt, dass seine Umschlagkante 28 nach oben und somit in Richtung der Hohlnadel 56 weist. Der innendurchmesser des Abschnitts 52c ist so bemessen, dass der Flüssigkeitsbeutel 20 in der dargestellten Ausrichtung gestützt wird und nicht ausweichen kann.

Zur Ausbringung der Flüssigkeit 38 aus dem Flüssigkeitsbeutel 20 wird die zweite Teileinheit 60 durch Kraftbeaufschlagung der Betätigungseinheit 40 an Fingerauflageflächen 50a, 60a tiefer in die erste Teileinheit 50 eingeschoben, so dass ein Innenvolumen des Aufnahmeraums 70 vermindert wird. Dies führt in der in Fig. 2b dargestellten Weise dazu, dass der Flüssigkeitsbeutel 20 zwischen dem Schutzabschnitt 58 und der Kolbenfläche 62 zusammengedrückt wird und sich der Druck im Flüssigkeitsbeutel 20 somit erhöht. Sobald eine Stauchung des ungeöffneten Flüssigkeitsbeutels 20 nicht mehr möglich ist, kann das Zusammendrücken der Teileinheiten 50, 60 erst fortgesetzt werden, wenn eine ausreichend hohe Betätigungskraft - etwa 20 Newton - aufgebracht wird, um den Sicherungsring 54b vom Einsatz 54 loszubrechen. Sobald dies geschieht, wird mit fortgesetzter Relativverlagerung der Teileinheiten 50, 60 der Schutzabschnitts 58 gegenüber den Bauteilen 52, 54, 56 nach oben verschoben.

Die Verlagerung des Schutzabschnitts 58 in der in Fig. 2c dargestellten Weise nach oben führt dazu, dass die Hohlnadel 56 mit ihrer Spitze 56a voran in den nachrückenden Flüssigkeitsbeutel 20 an dessen umgeschlagener Kante 28 eindringt. Die fortgesetzte Relativbewegung der Teileinheiten 50, 60 führt zur Volumenverringerung des Aufnahmeraums 70 und damit des innenraums des Flüssigkeitsbeutel 20. Die Flüssigkeit 38 wird durch die Hohlnadel 56 und den Flüssigkeitskanal 54a hindurch zur Auslassöffnung 52b gefördert und bildet dort einen Sprühstrahl 36. Durch die konische Gestaltung der Hohlnadel 56 dichtet diese die Einstichöffnung an der umgeschlagenen Kante 28 des Flüssigkeitsbeutels 20 gut ab. Dennoch aus dem Flüssigkeitsbeutel 20 hinaus in den Aufnahmeraum 70 gelangende Flüssigkeit kann durch den Schlitz 56c in die Hohlnadel 56 einströmen und somit ebenfalls ausgebracht werden.

Während der fortgesetzten Betätigung wird der Flüssigkeitsbeutel 20 derart weit zusammengedrückt, dass die Hohlnadel 56 im Bereich der Schweißnaht 22 wieder aus dem Flüssigkeitsbeutel 20 austritt, wie in Fig. 2d dargestellt ist. Ihre Spitze rückt dann in die schachtartige Vertiefung 62a der zweiten Teileinheit 60 ein, so dass sie Fortsetzung des Austragvorgangs nicht behindert. Auch wenn in dieser letzten Phase die Flüssigkeit aus dem Flüssigkeitsbeutel 20 nicht mehr unmittelbar durch die Öffnung 56b in die Hohlnadel 56 eintreten kann, so ist doch aufgrund des Längsschlitzes 56c gewährleistet, dass die Flüssigkeit aus dem Flüssigkeitsbeutel 20 in die Hohlnadel 56 und somit zur Auslassöffnung 52b gelangen kann.

Die Fig. 3a bis 3d zeigen eine zweite Ausführungsform einer erfindungsgemäßen Austragvorrichtung. Bei dieser zweiten Ausführungsform ist die Betätigungseinheit 140, die wiederum zur Ausbringung der Flüssigkeit aus einem Flüssigkeitsbeutel 20 vorgesehen ist, einfacher als bei der ersten Ausführungsform der Fig. 2a bis 2d ausgebildet. Die erste Teileinheit 150 besteht wiederum aus einem hülsenförmigen Bauteil 152, in welches ein Einsatz 154 eingesetzt ist. Diese zweite Ausführungsform weist allerdings weder eine Hohlnadel 56 noch einen verlagerbaren Schutzabschnitt 58 auf. Der Flüssigkeitskanal 154a erstreckt sich unmittelbar vom Aufnahmeraum 170 bis zur Auslassöffnung 152b.

Die zweite Teileinheit 160 ähnelt der Gestaltung der ersten Ausführungsform, weist jedoch keine Vertiefung 62a auf.

Bei der gezeigten zweiten Ausführungsform ist vorgesehen, dass der Flüssigkeitsbeutel 20 nicht durch ein Öffnungsmittel wie der beschriebenen Hohlnadel 56 geöffnet wird, sondern ausschließlich durch seinen lnnendruck. Es kann allerdings vorgesehen sein, dass der Flüssigkeitsbeutel 20 gezielt vorgesehene Schwächungen aufweist, um ein reproduzierbares Öffnungsverhalten zu bewirken. Vorliegend ist vorgesehen, dass die in Richtung des Auslasskanals 154a weisende Seite des Flüssigkeitsbeutels 20 mit einer geschwächten Längsnaht 22 versehen ist, deren Versagen vor dem Versagen der Quernähte 24 zu erwarten ist.

Ausgehend von dem Zustand der Fig. 3a wird auch bei der zweiten Gestaltung mittels Kraftbeaufschlagung auf Fingerauflageflächen 150a, 160a eine Druckerhöhung im Flüssigkeitsbeutel 20 bewirkt. Sobald die Betätigungskraft ausreichend hoch ist (ca. 20 Newton) und dadurch der Innendruck in der Flüssigkeit ausreichend hoch ist (ca. 4 bar), versagt die Längsnaht 22, so dass der Flüssigkeitsbeutel 20 an seinem oberen Ende aufreißt, wie in Fig. 3c mit dem Bezugszeichen 22' angedeutet ist. Damit ist der Weg für das Medium zur Auslassöffnung 152b frei. Im Zuge der fortgesetzten Verlagerung der Teileinheiten 160, 150 bis zum Zustand der Fig. 3d wird somit ein Austrag der Flüssigkeit durch die Auslassöffnung 152b bewirkt.

Diese zweite Gestaltung ist gegenüber der ersten Gestaltung in der Herstellung deutlich günstiger, wobei der Austrag der gesamten Flüssigkeit 38 aus dem Flüssigkeitsbeutel 20 bei der ersten Gestaltung in vollständigerem- Maße gewährleistet ist. Abhängig vom jeweiligen Anwendungszweck kann eine der beiden Gestaltungen gewählt werden.

## Patentansprüche

1. Austragvorrichtung für pharmazeutische Flüssigkeiten mit
- einer Betätigungseinheit (40; 140) zur manuellen Bewirkung eines Austragvorgangs und
- einem Flüssigkeitsspeicher (20), in dem die pharmazeutische Flüssigkeit (38) vor dem Austragvorgang gelagert ist, wobei
- eine erste Teileinheit (50; 150) der Betätigungseinheit (40; 140) mit einer Auslassöffnung (52b; 152b) versehen ist und
- eine zweite Teileinheit (60; 160) der Betätigungseinheit (40; 140) zur Bewirkung des Austragvorgangs gegenüber der ersten Teileinheit (50; 150) verlagerbar ist,
**dadurch gekennzeichnet, dass**
- die beiden Teileinheiten (50, 60; 150, 160) der Betätigungseinheit (40; 140) gemeinsam einen Aufnahmeraum (70; 170) begrenzen, von dem aus ein Flüssigkeitskanal (54a; 154a) zur Auslassöffnung (52b; 152b) führt,
- der Flüssigkeitsspeicher (20) zur Anordnung im Aufnahmeraum (70; 170) vorgesehen und als Flüssigkeitsbeutel (20) ausgebildet ist, dessen Wandungen (30, 32, 34) als Folienwandungen (30, 32, 34) ausgebildet sind, die einen mit der Flüssigkeit (38) befüllten Innenraum begrenzen, und
- die Betatigungseinheit (40; 140) und der Flüssigkeitsbeutel (20) derart aufeinander abgestimmt sind, dass durch eine Relativverlagerung der Teileinheiten (50, 60; 150, 160) der Betätigungseinheit (40; 140) zueinander
- ein Öffnen des Flüssigkeitsbeutels (20) und
- eine Volumenreduzierung des Aufnahmeraums (70) mit daraus resultierender Volumenreduzierung des Flüssigkeitsbeutels (20) und ein Ausbringung der Flüssigkeit (38) durch die Auslassöffnung (52b, 152) bewirkt wird.

2. Austragvorrichtung nach Anspruch 1,
**dadurch gekennzeichnet, dass**
ein Kontaktbereich, in dem die beiden Teileinheiten (50, 60; 150, 160) aneinander anliegen, flüssigkeitsdicht ausgebildet ist, so dass Flüssigkeit aus dem Aufnahmeraum (70; 170) ausschließlich über die Auslassöffnung (52b; 152b) in eine die Austragvorrichtung umgebende Atmosphäre gelangen kann.

3. Austragvorrichtung nach Anspruch 1 oder 2,
**dadurch gekennzeichnet, dass**
- der Flüssigkeitsbeutel (20) eine längliche Form aufweist,
- der Durchmesser des Flüssigkeitsbeutels (20) zwischen 3 mm und 10 mm beträgt, vorzugsweise zwischen 4 mm und 6 mm,
- die Länge des Flüssigkeitsbeutels (20) zwischen 10 mm und 30 mm beträgt, vorzugsweise zwischen 15 mm und 25 mm, und/oder
- das Innenvolumen des Flüssigkeitsbeutels (20) unter 1000 µl beträgt, vorzugsweise zwischen 100 und 500 µl beträgt.

4. Austragvorrichtung nach einem der vorstehenden Ansprüchen, **dadurch gekennzeichnet, dass**
die den Innenraum des Flüssigkeitsbeutels umgebende Folienwandung (30, 32, 34) von einheitlicher Art ist, wobei vorzugsweise der Flüssigkeitsbeutel (20) als in einem kontinuierlichen Verfahren hergestellter Schlauchbeutel ausgebildet ist.

5. Austragvorrichtung nach einem der vorstehenden Ansprüche, **dadurch gekennzeichnet, dass**
eine Folienwandungen (30, 32, 34) des Flüssigkeitsbeutels diffusionsdicht ausgebildet ist, insbesondere durch
- eine Beschichtung (30b) der Folienwandung (30a), insbesondere mit Metall, Keramik und/oder
- die Ausbildung der Folienwandung (30, 32, 34) oder einer Schicht der Folienwandung (32, 34, 36) als Folie (30b) aus Metall, insbesondere Aluminium, oder aus Ethylen-Vinyl-Alkohol-Copolymer (EVOH).

6. Austragvorrichtung nach einem der vorstehenden Ansprüche, **dadurch gekennzeichnet, dass**
die Betätigungseinheit (140) und der Flüssigkeitsbeutel (20) derart aufeinander abgestimmt sind, dass durch eine Relativverlagerung der beiden Teileinheiten (150, 160) der Betätigungseinheit (140) eine Druckerhöhung im Flüssigkeitsbeutel (20) bewirkt werden kann, die bei ausreichender Kraftbeaufschlagung der beiden Teileinheiten (150, 160) gegeneinander ein Platzen des Flüssigkeitsbeutels (20), insbesondere im Bereich einer Folienwandung oder einer Naht (22), verursacht.

7. Austragvorrichtung nach Anspruch 1 bis 5,
**gekennzeichnet durch**
ein scharfkantiges Offnungsmittel (56a) zum Öffnen des Flüssigkeitsbeutels (20).

8. Austragvorrichtung nach Anspruch 7,
**dadurch gekennzeichnet, dass**
das Öffnungsmittel (56a) durch eine an ihrem distalen Ende offene und mit dem Flüssigkeitskanal (54a) verbundene Hohlnadel (56) gebildet ist, wobei vorzugsweise die Hohlnadel (56)
- zumindest in einem Teilabschnitt eine seitliche angeordnete Öffnung (56c) aufweist und/oder
- zumindest über einen Teilabschnitt sich zum distalen Ende hin verjüngend ausgebildet ist.

9. Austragvorrichtung nach Anspruch 7 oder 8,
**dadurch gekennzeichnet, dass**
dem Öffnungsmittel (56a) ein gegenüber dem Öffnungsmittel (56a) relativ verlagerbarer Schutzabschnitt (58) zugeordnet ist, der in einer Sicherungsstellung verhindert, dass das Öffnungsmittel (56a) mit dem Flüssigkeitsbeutel (20) in Kontakt gelangt, und der in einer Funktionsstellung das Öffnungsmittel (56a) freigibt.

10. Austragvorrichtung nach Anspruch 9,
**dadurch gekennzeichnet, dass**
der Schutzabschnitt (58) derart angeordnet und/oder ausgebildet ist, dass er durch eine Kraftbeaufschlagung der Teileinheiten (50, 60) der Betätigungseinheit (40) aus der Sicherungsstellung in die Funktionsstellung verlagerbar ist, wobei vorzugsweise vorgesehen ist, dass diese Kraftbeaufschlagung über den Flüssigkeitsbeutel (20) auf den Schutzabschnitt (58) übertragen wird.

11. Austragvorrichtung nach einem der vorstehenden Ansprüche,
**dadurch gekennzeichnet, dass**
die Betätigungseinheit (40; 140) und/oder der Flüssigkeitsbeutel (20) derart ausgebildet ist, dass ein Öffnen des Flüssigkeitsbeutels (20) bewirkt wird, wenn die Teileinheiten (50, 60; 150, 160) mit einer Betätigungskraft von mindestens 10 Newton, vorzugsweise mir einer Betätigungskraft zwischen 20 Newton und 30 Newton, gegeneinander gedrückt werden, wobei vorzugsweise vorgesehen ist, dass der Schutzabschnitt (58) bis zum Erreichen dieser Betätigungskraft über mindestens einen eine Sollbruchstelle aufweisenden Sicherungsabschnitt (54b) in seiner Sicherungsstellung gehalten wird.

12. Flüssigkeitsbeutel (20) für eine Austragvorrichtung nach einem der vorstehenden Ansprüche,
**dadurch gekennzeichnet, dass**
die Wandungen (30, 32, 34) des Flüssigkeitsbeutels (20) als Folienwandungen (30, 32, 34) ausgebildet sind, die einen mit einer pharmazeutischen Flüssigkeit (38) befüllten Innenraum begrenzen, wobei der Innenraum ein Volumen von maximal 1000 µl, insbesondere zwischen 100 µl und 500 µl aufweist und wobei der In~ nenraum mit der pharmazeutischen Flüssigkeit (38), insbesondere mit einem Impfstoff oder mit einem Schmerzmittel, gefüllt ist.

13. Flüssigkeitsbeutel nach Anspruch 12,
**dadurch gekennzeichnet, dass**
- der Flüssigkeitsbeutel (20) eine längliche Form aufweist,
- der Durchmesser des Flüssigkeitsbeutels (20) zwischen 3 und 10 mm beträgt, vorzugsweise zwischen 4 und 6 mm beträgt, und/oder
- die Länge des Flüssigkeitsbeutels (20) zwischen 10 mm und 30 mm beträgt, vorzugsweise zwischen 15 mm und 25 mm beträgt.

14. Flüssigkeitsbeutel nach einem der Ansprüche 12 oder 13,
**dadurch gekennzeichnet, dass**
der Flüssigkeitsbeutel (20) als Schlauchbeutel ausgebildet ist.

15. Mehrzahl von Flüssigkeitsbeuteln nach einem der Ansprüche 12 bis 14,
**dadurch gekennzeichnet, dass**
die Flüssigkeitsbeutel (20) einen zusammenhängenden Streifen (10) bildet, wobei zwischen den einzelnen Flüssigkeitsbeuteln (20) werkzeuglos trennbare Perforationen (26) vorgesehen sind.
